# EUROPÄISCHE PATENTSCHRIFT

(11) **EP 1 981 965 B1**
(45) Veröffentlichungstag und Bekanntmachung des Hinweises auf die Patenterteilung: **14.10.2015**
(21) Anmeldenummer: 07703324.9
(22) Anmeldetag: 07.02.2007
(51) Int. Cl.: C12N 5/00

(54) **VERFAHREN ZUR KULTIVIERUNG BIOLOGISCHER ZELLEN**
PROCESS FOR CULTURING BIOLOGICAL CELLS
PROCÉDÉ DE CULTURE DE CELLULES BIOLOGIQUES

(30) Priorität: 10.02.2006 DE 102006006269
(43) Veröffentlichungstag der Anmeldung: 22.10.2008
(73) Patentinhaber: Fraunhofer-Gesellschaft zur Förderung der angewandten Forschung e.V., 80686 München (DE)
(72) Erfinder: FUHR, Günter, R., 13187 Berlin (DE); VON BRIESEN, Hagen, 65510 Hünstetten (DE); GORJUP, Erwin, 66111 Saarbrücken (DE); KRUSE, Charli, 23923 Herrnburg (DE)
(74) Vertreter: Hertz, Oliver
(86) Internationale Anmeldenummer: PCT/EP2007/001041
(87) Internationale Veröffentlichungsnummer: WO 2007/090629

(56) Entgegenhaltungen:
- EP-A- 0 085 573
- WO-A-01/98517
- WO-A-97/21801
- WO-A-02/088329
- WO-A-2005/113750
- US-A- 4 514 500
- GALINDO ENRIQUE ET AL: "Study of drop and bubble sizes in a simulated mycelial fermentation broth of up to four phases" BIOTECHNOLOGY AND BIOENGINEERING, Bd. 69, Nr. 2, 20. Juli 2000 (2000-07-20), Seiten 213-221, XP002426886 ISSN: 0006-3592

## Beschreibung

Die Erfindung betrifft ein Verfahren zur Kultivierung biologischer Zellen in einer nicht-festen Kultivierungsumgebung, insbesondere ein Verfahren zur Zellkultivierung, bei dem biologische Zellen an mindestens einer Grenzfläche zwischen flüssigen und/oder gasförmigen Kultivierungsphasen angeordnet sind.

Die Kultivierung (Wachstum, Vermehrung und/oder Differenzierung) biologischer Zellen erfolgt in der Praxis typischerweise auf festen Oberflächen, wie z. B. auf dem Boden einer Petrischale oder auf anderen Kultivierungssubstraten. Beim Wachstum einer in vitro-Zellkultur bilden sich auf der festen Oberfläche Zellmonoschichten oder schichtförmige Gewebeformationen, deren Form der Oberflächengestalt entspricht. Nachteilig an der Kultivierung auf festen Oberflächen ist, dass sich die Zellen im Zellverband unter geometrischen Bedingungen entwickeln, die durch die Oberflächengestalt des Kultivierungssubstrates bestimmt sind und in der Regel nicht den natürlichen Bedingungen entsprechen. Mit der Prägung der Zellkultur durch die Oberflächengestalt wird die Ausbildung natürlicher Strukturen im Zellverband behindert. Ein weiterer Nachteil der *in* vitro-Kultivierung auf einer festen Oberfläche ergibt sich aus der molekularen Anhaftung der Zellen auf der Oberfläche. Es werden durch Haftproteine, z. B. der Integrinfamilie adhärente Bindungen zwischen den Zellen und der Oberfläche gebildet, die bei Manipulationen an der Zellkultur gelöst werden müssen. Beispielsweise erfordert ein Transfer von Zellen auf ein anderes Substrat eine enzymatische Behandlung, z. B. mit Trypsin oder ein mechanisches Abkratzen der Zellen von der Oberfläche. In beiden Fällen sind ein erhebli-cher Stress für die Zellen und ein Zellverlust nicht zu vermeiden.

Zur Vermeidung der Prägung der Zellkultur durch die Oberflächengestalt eines festen Substrates wird in DE 10 2004 025 086.3 die Verwendung von einem Wirts-Ei als Inkubator für *in* vitro-Kulturen beschrieben. Vorteilhafterweise werden damit Kultivierungsbedingungen geschaffen, welche in Bezug auf die geometrischen Bedingungen und die Nährstoffversorgung den natürlichen Wachstumsbedingungen am nächsten kommen. Nachteilig an dieser Technik ist jedoch, dass die Umgebungsbedingungen bei der Zellkultivierung durch die Eigenschaften des verwendeten Wirts-Ei vorgegeben sind.

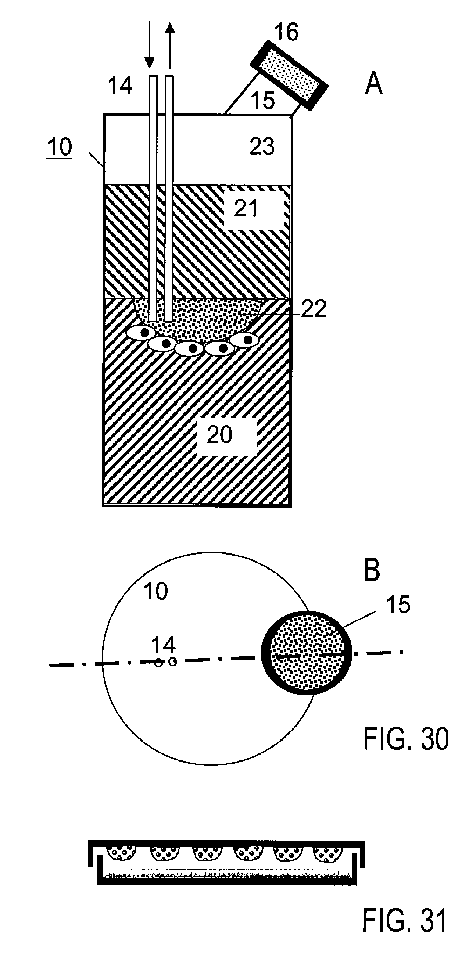

Aus der Praxis der Kultivierung von Stammzellen ist bekannt, einzelne Zellen oder Zellgruppen in hängenden Tropfen eines flüssigen Kultivierungsmediums zu kultivieren, wie es schematisch in Figur 31 gezeigt ist. Vorteilhafterweise sind im hängenden Tropfen Bedingungen gegeben, die ähnlich den geometrischen Bedingungen des Wachstums z. B. in einem Ei sind. Die Zellen können sich im flüssigen Kultivierungsmedium auf der gekrümmten inneren Oberfläche des hängenden Tropfens frei bewegen und Zellgruppen oder Zellverbände bilden. Ein entscheidender Nachteil dieser Technik besteht jedoch in der beschränkten Größe der hängenden Tropfen. Die Tropfen können nur Durchmesser von bis zu einigen Millimetern aufweisen, so dass entsprechend die Kultivierungszeit und die Größe der kultivierten Zellgruppen limitiert sind.

Es ist bekannt, Zellkulturen in Bioreaktoren (Diffusionsreaktoren, Fermenter) zu halten, um Substanzen zu gewinnen, die von den Zellen produziert werden (siehe zum Beispiel G.M. Zijlstra et al. in "Current opinion in biotechnology", Band 9, 1998, S. 171-176; C.T. MacLeod et al. in "Process Biochemistry", Band 40, 2005, S. 1799-1805; A.J. Daugulis in "Current opinion in biotechnology", Band 8, 1997, S. 169-174; M.G. Antov et al. in "Enzyme Microbial. Technol.", Band 28, 2001, S. 467-472; und K.G. Clarke et al. in "Biochem. Eng. J." Band 28, 2006, S. 237-242). Typischerweise ist in einem Bioreaktor ein flüssiges Mehrphasensystem aus mehreren nichtmischbaren Phasen enthalten, wobei Substanzen, die von den Zellen in einer ersten Phase produziert werden, in eine benachbarte Phase diffundieren und von dort abgeführt werden. Entsprechend wird eine Rückwirkung der Substanzen auf die Zellen in der ersten Phase vermieden. Die Anwendung der herkömmlichen Bioreaktoren ist auf biotechnologischer Funktionen, wie zum Beispiel die Substanzproduktion beschränkt. Mit einem Bioreaktor können Kultivierungsbedingungen eingestellt werden, unter denen die Zellen lebensfähig sind und die gewünschte Substanz produzieren. Die herkömmlichen Bioreaktoren sind jedoch nicht für Kultivierungsaufgaben der Zellbiologie geeignet, wie zum Beispiel die oben genannte Kultivierung von Zellen im Zellverband.

Das Wachstum biologischer Zellen an suspendierten Tropfen wird z. B. in EP 085 573 A2 und US 4 514 500 beschrieben. Die Fermentation biologischer Zellen unter Einwendung von Tropfen-Suspensionen wird z. B. von E. Galindo et al. im "Bio-technology and Bioengineering", Band 69, 200, S. 213-221 beschrieben.

Die Aufgabe der Erfindung ist es, ein verbessertes Verfahren zur Kultivierung biologischer Zellen bereitzustellen, mit dem die Nachteile der herkömmlichen Kultivierungstechniken überwunden werden. Das Verfahren soll insbesondere eine Zellkultivierung unter Bedingungen ermöglichen, die nicht durch die Gestalt oder Größe eines Kultivierungsgefäßes beeinflusst sind und gleichzeitig eine Anpassung an natürliche Bedingungen des Wachstums, der Vermehrung und/oder der Differenzierung von Zellen ermöglichen.

Diese Aufgabe wird durch ein Zellkultivierungsverfahren mit den Merkmalen von Anspruch 1 gelöst. Vorteilhafte Ausführungsformen und Anwendungen der Erfindung ergeben sich aus den abhängigen Ansprüchen.

Verfahrensbezogen wird die Aufgabe der Erfindung insbesondere durch die technischen Lehre gelöst, bei einer Kultivierung biologischer Zellen an einer gekrümmten Grenzfläche zwischen zwei flüssigen, nicht mischbaren, nicht-festen Kultivierungsphasen vorbestimmte Kultivierungsbedingungen bereitzustellen, indem die Eigenschaften der an die Grenzfläche angrenzenden Kultivierungsphasen gezielt eingestellt werden. Mindestens eine innere Kultivierungsphase wird von einer äußeren Kultivierungsphase vollständig eingeschlossen. Vorteilhafterweise werden Kultivierungsbedingungen eingestellt, die zur Beeinflussung der Differenzierung der Zellen geeignet sind. Es wird auch eine Kultivierungseinrichtung mit einem Kulturgefäß beschrieben, das mindestens zwei nicht-feste Kultivierungsphasen enthält, zwischen denen eine vorbestimmte Grenzfläche gebildet ist.

Die Kultivierungsphasen werden in dem Kulturgefäß angeordnet, wobei zunächst die äußere Kultivierungsphase in das Kulturgefäß eingefüllt und anschließend die innere Kultivierungsphase injiziert wird und die Kultivierungsphasen-Einstellung in dem Kulturgefäß vor oder während einer Ablage der Zellen an der Grenzfläche erfolgt. Die Kultivierungsphasen-Einstellung umfasst eine Beeinflussung der geometrischen Eigenschaften der Kultivierungsphasen, so dass die Grenzfläche zwischen den Kultivierungsphasen eine vorbestimmte Form erhält, und eine Anordnung der Kultivierungsphasen relativ zueinander, so dass die Grenzfläche eine vorbestimmte Ausdehnung aufweist, wobei mit einer Injektionseinrichtung in die Kultivierungsphasen in der Kultivierungseinrichtung Zusatzstoffe eingeführt werden, mit denen die Massendichte der Kultivierungsphasen verändert und die Grenzfläche in der Kultivierungseinrichtung positioniert und in ihrer Form stabilisiert wird. Die Kultivierung umfasst eine Differenzierung der biologischen Zellen durch Signalfaktoren und/oder Differenzierungsfaktoren, die in mindestens einer der Kultivierungsphasen enthalten sind.

Durch die gezielte Einstellung der Kultivierungsphasen wird erfindungsgemäß vorteilhafterweise mindestens eine Grenzfläche geformt, an der sich die biologischen Zellen während der Zellkultivierung anordnen. Die mindestens eine Grenzfläche dient vorzugsweise als Substrat. Im Unterschied zur herkömmlichen Kultivierung an festen Oberflächen sind die Zellen von der Grenzfläche leicht ablösbar. Die Nachteile der Oberflächenanhaftung z. B. für eine Zelltransfer werden ausgeschlossen. Im Gegensatz zur herkömmlichen Kultivierung von Zellen in einem Wirts-Ei werden die Kultivierungsbedingungen durch die Einstellung der Kultivierungsphasen bereitgestellt. Die Beschränkung auf die geometrischen und stofflichen Kultivierungsbedingungen wird überwunden, die bei der herkömmlichen Technik durch die natürliche Eiform vorgegeben waren. Entsprechend können auch die Größenbeschränkungen der herkömmlichen Kultivierung in hängenden Tropfen überwunden werden.

Vorzugsweise ist die zur Ausführung des Verfahrens verwendete Kultivierungseinrichtung künstlich hergestellt, d.h. aus Komponenten zusammengesetzt, wie sie in der Natur nicht zusammenwirken.

Der Begriff "Kultivierungsphase" bezeichnet hier einen Bereich im Kulturgefäß, der stofflich homogen und gegenüber der Umgebung abgegrenzt ist. Die Kultivierungsphase weist eine bestimmte Volumenform auf. Die Kultivierungsphase wird durch ein nicht-festes Medium (sog. Kultivierungsmedium) gebildet, das z. B. eine einzige chemische Verbindung oder eine chemische Substanzzusammensetzung umfasst. Die nicht-feste Kultivierungsphase besteht aus einem fließfähigen, insbesondere flüssigen Medium. Kultivierungsphasen dienen der Formung der mindestens einen Grenzfläche und ggf. der Zufuhr von Nährstoffen zu den Zellen an der Grenzfläche, wie z. B. die Zufuhr von Wachstumsfaktoren durch die flüssige Kultivierungsphase. Eine Zufuhr von Sauerstoff oder Kohlendioxid durch eine gasförmige Kultivierungsphase wird auch beschrieben. Eine erste Kultivierungsphase kann eine oder mehrere weitere Kultivierungsphasen vollständig einschließen oder umhüllen. Entsprechend kann eine äußere, einschließende Kultivierungsphase eine Umgebungslösung sein, in der die mindestens eine weitere Kultivierungsphase eingebettet ist.

Der Begriff "Kultivierungsbedingungen" bezeichnet die Umgebungsbedingungen der Zellen an der mindestens einen Grenzfläche zwischen den Kultivierungsphasen, insbesondere die Bedingungen des Wachstums, der Vermehrung und/oder der Differenzierung. Bevorzugt werden bei der erfindungsgemäßen Kultivierungsphasen-Einstellungen gezielt vorbestimmte Kultivierungsbedingungen gebildet, die sich auf eine Differenzierung der Zellen auswirken (Differenzierungsbedingungen).

Der Begriff "Kultivierungsphasen-Einstellung" bezeichnet hier insbesondere die Einstellung vorbestimmter geometrischer, physikalischer und/oder chemischer Eigenschaften der Kultivierungsphasen. Die Einstellung der Kultivierungsphasen bedeutet eine gerichtete Veränderung mindestens einer der genannten Eigenschaften, so dass die gewünschten Kultivierungsbedingungen gegeben sind.

Vorteilhafterweise wird mit der Erfindung im Unterschied zu den herkömmlichen Kultivierungstechniken eine Differenzierung und/oder geordnete (vorbestimmte) Gewebeentwicklung von Zellen an Grenzflächen zwischen verschiedenen, nicht-festen Kultivierungsphasen realisiert. Es können insbesondere die Kultivierungsbedingungen so eingestellt werden, dass in unmittelbarer Nachbarschaft verschiedene Zell- und/oder Gewebetypen differenziert werden und/oder wachsen.

Vorteilhafterweise kann die Erfindung mit einem breiten Spektrum verschiedener Zellarten realisiert werden. Besonders bevorzugt erfolgt die Zellkultivierung mit tierischen Zellen, insbesondere Zellen höherer Tierklassen, wie zum Beispiel Zellen von Fischen, Säugetieren oder Vögeln, oder mit humanen Zellen. Alternativ können auch Pflanzenzellen der erfindungsgemäßen Zellkultivierung unterzogen werden.

Gemäß der Erfindung umfasst die Kultivierungsphasen-Einstellung eine Beeinflussung der geometrischen Eigenschaften der Kultivierungsphasen, so dass die mindestens eine Grenzfläche zwischen den Kultivierungsphasen eine vorbestimmte Form erhält. Da gemäß der Erfindung eine gekrümmte Grenzfläche geformt wird, wird vorteilhafterweise ein gekrümmtes Substrat für die Zellkultur gebildet, wie es sich bei *in vi*vo-Kulturen in der Natur herausgebildet hat. Die Zellkultivierung kann in einem gekrümmten Zellverbund erfolgen, dessen Geometrie an natürliche Vorbilder angepasst ist.

Die Einstellung einer bestimmten Form der Grenzfläche zwischen den Kultivierungsphasen kann eine freie Formung unter der Wirkung der Oberflächenspannung (eingestellt insbesondere mit der Viskosität und der Größe der Kultivierungsphase) oder eine erzwungene Formung durch die Bereitstellung einer bestimmten Wandform des Kulturgefäßes umfassen, in dem die Kultivierungsphasen angeordnet sind.

Gemäß der Erfindung umfasst die Kultivierungsphasen-Einstellung des Weiteren eine Anordnung der Kultivierungsphasen relativ zueinander derart, dass die Grenzfläche eine vorbestimmte Ausdehnung (Größenmaß) ausweist. Vorteilhafterweise kann das Volumen der *in* vitro-Zellkultur in Abhängigkeit von den konkreten Anforderungen der Kultivierungsaufgabe gesteuert werden. Die Kultivierungsphasen können mit Volumen von µl-Bereich bis zum Liter-Bereich gesteuert werden. Die *in* vitro-Systeme sind skalierbar.

Gemäß einer vorteilhaften Ausführungsform der Erfindung umfasst die Kultivierungsphasen-Einstellung eine Steuerung der chemischen Zusammensetzung der Kultivierungsphasen. Die erfindungsgemäß eingestellten Kultivierungsphasen werden mit vorbestimmten flüssigen Kultivierungsmedien gebildet. Mit der Steuerung der chemischen Zusammensetzung können erstens die physikalische Wechselwirkung (z. B. Mischbarkeit) benachbarter Kultivierungsphasen und damit die mindestens eine Grenzfläche (geometrischer Effekt) oder zweitens die chemischen Bedingungen der Kultivierung (chemischer Effekt) beeinflusst werden. Vorteilhafterweise können dabei in den Kultivierungsphasen insbesondere chemische Gradienten erzeugt und stabilisiert werden, wie sie bspw. bei der Embryogenese von Bedeutung sind. Des Weiteren kann die chemische Zusammensetzung benachbarter Kultivierungsphasen so gewählt werden, dass an der Grenzfläche, wo die Zellkultivierung erfolgt, chemische Reaktionen zwischen den Kultivierungsphasen vorbestimmte Wirkstoffe liefern, mit denen die Zellkultivierung beeinflusst wird.

Gemäß einer bevorzugten Variante umfasst die Kultivierungsphasen-Einstellung auch die Zufuhr von vorbestimmten Zusatzstoffen in die Kultivierungsphasen, um die geometrischen, physikalischen oder chemischen Eigenschaften der Kultivierungsphasen zu steuern. Es werden vorzugsweise Zusatzstoffe zugesetzt, mit denen die Dichte, die Viskosität und/oder die Mischbarkeit der Kultivierungsphasen einstellbar sind. Vorteilhafterweise ist eine breite Vielfalt von Zusatzstoffen verfügbar, mit denen die Mischbarkeit von flüssigen Kultivierungsphasen unterbunden oder behindert wird und die inert für die biologischen Zellen sind, wie z. B. Polymerverbindungen, Gele, Öle, insbesondere zellverträgliche Silikonöle, Zuckerverbindungen, insbesondere langkettige Dextrane, Metrizamid etc., oder Glyzerol.

Zusatzstoffe, welche die Viskosität einer Kultivierungsphase einstellen, wie z. B. hochmolekulare Zucker, welche die Osmolarität vorzugsweise nicht ändern, oder Gelzusätze, wie z. B. Agar ermöglichen vorteilhafterweise eine wesentliche Beschränkung oder komplette Verhinderung einer Relativbewegung verschiedener Kultivierungsphasen infolge der Gravitation. Durch Zusatzstoffe, welche die Viskosität erhöhen, können bspw. gasförmige Kultivierungsphasen (Gasblasen) in flüssigen Kultivierungsphasen stabilisiert werden.

Mit Zusatzstoffen, welche die Massendichte einer Kultivierungsphase steuern, werden benachbarte Kultivierungsphasen zueinander angepasst. Es kann bspw. eine Kultivierungsphase leichter, mit der gleichen Massendichte oder schwerer als eine umgebende Kultivierungsphase eingestellt werden. Vorteilhafterweise können damit die für die Zellkultur vorgesehenen Grenzflächen exakt positioniert und in ihrer Form stabilisiert werden. Dies ermöglicht eine reproduzierbare und genaue Einstellung der chemischen Eigenschaften der Kultivierungsphasen, z. B. durch die Zufuhr von vorbestimmten Nährmedien.

Erfindungsgemäß kann ein langsames Wandern von Kultivierungsphasen durch eine Umgebungslösung oder eine äußere Kultivierungsphase vorgesehen sein. Dies ist besonders für die Kultivierung von Zellen in vertikalen Gradienten von Vorteil.

Zusatzstoffe zur Unterbindung der Mischbarkeit können gemäß einer weiteren Variante der Erfindung Membranen an der Grenzfläche zwischen zwei benachbarten Kultivierungsphasen bilden, wie z. B. Lipidmembranen, Proteinmembranen oder Polymermembranen.

Die Kultivierungseinrichtung weist eine Injektionseinrichtung auf, mit der flüssige oder gasförmige Substanzen in das Kulturgefäß einführbar sind. Vorteilhafterweise kann die Injektionseinrichtung zur Zuführung von Nährmedien und/oder Zusatzstoffen, zur Formung der Flüssigkeitsphasen oder zur Stabilisierung der Flüssigkeitsphasen verwenden werden. Gemäß hier beschriebenen Varianten umfasst die Injektionseinrichtung mindestens einen Flüssigkeitsinjektor und/oder mindestens einen Gasinjektor, der jeweils eine von einem Substanzreservoir in das Kulturgefäß gerichtete Injektionsleitung aufweist. Es ist vorzugsweise für jede Substanz (Nährmedium oder Zusatzstoff) ein separater Injektor vorgesehen. Gemäß einer weiteren Variante umfasst die Injektionseinrichtung einen Suspensionsinjektor, mit dem Suspensionsflüssigkeiten in das Kulturgefäß einführbar sind. Eine Suspensionsflüssigkeit umfasst bspw. eine Zellsuspension oder eine Suspension mit synthetischen Partikeln.

Für die erfindungsgemäße Kultivierungsphasen-Einstellung weist die Kultivierungseinrichtung vorzugsweise mindestens eine der folgenden Komponenten auf. Wenn eine Temperierungseinrichtung und/oder eine Befeuchtungseinrichtung vorgesehen ist, können vorteilhafterweise bestimmte Kultivierungsbedingungen, wie z. B. die Temperatur und/oder die Umgebungsfeuchte für alle Kultivierungsphasen gemeinsam eingestellt werden.

Wenn die Kultivierungseinrichtung mit mindestens einer Messeinrichtung ausgestattet ist, können die korrekte Deposition biologischer Zellen an der mindestens einen Grenzfläche, der Fortschritt der Zellkultivierung und/oder Transfer von Zellen, wie z. B. die Entnahme von Zellen aus dem Kulturgefäß überwacht werden. Die Kultivierungsphasen-Einstellung kann in Abhängigkeit vom Messwerten der Messeinrichtung gesteuert werden. Die Messeinrichtung umfasst vorzugsweise mindestens eine Sonde, die im Kulturgefäß in mindestens einer der Kultivierungsphasen angeordnet ist und bspw. einen Temperatursensor, einen chemischen Sensor oder einen pH-Sensor umfasst. Mit der Sonde kann bspw. in der Nähe der Grenzfläche die Temperatur, eine Ionenkonzentration, z. B. die Konzentration von zweiwertigen Ionen (z. B. Ca) oder pH-Wert gemessen werden. In Abhängigkeit von dem mindestens einen Messwert können geometrische, physikalische oder chemische Eigenschaften der Kultivierungsphase verändert werden.

Ein wichtiger Vorteil der Erfindung besteht in der Variabilität bei der Kombination verschiedener Kultivierungsphasen. Wenn die Zellen gemäß einer weiteren, hier beschriebenen Verfahrensvariante an einer Grenzfläche zwischen einer flüssigen und einer gasförmigen Kultivierungsphase kultiviert werden, können sich Vorteile für eine schnelle Nährstoffzufuhr aus der gasförmigen Kultivierungsphase ergeben. Die Zellen sind an der Grenzfläche vorzugsweise so angeordnet, dass sie vollständig von der Substanz der flüssigen Kultivierungsphase eingeschlossen sind. Auf der zur gasförmigen Kultivierungsphase weisenden Seite sind die Zellen durch einen Flüssigkeitsfilm bedeckt.

Eine gasförmige Kultivierungsphase kann allseits von der flüssigen Kultivierungsphase umgeben sein. Die gasförmige Kultivierungsphase bildet in der flüssigen Umgebung eine allseits geschlossene Blase, z. B. in Form eines Ellipsoids. Bei dieser Variante wird eine konkav gekrümmte Grenzfläche mit Kultivierungsbedingungen ähnlich wie bei der Kultivierung im natürlichen Ei oder in hängenden Tropfen bereitgestellt, wobei jedoch die Form und Größe der Grenzfläche erfindungsgemäß gezielt steuerbar ist. Beispielsweise kann die Viskosität der flüssigen Kultivierungsphase ausreichend hoch eingestellt werden, dass die gasförmige Kultivierungsphase für die Dauer der Zellkultivierung stabil in der flüssigen Kultivierungsphase gehalten wird. Die gasförmige Kultivierungsphase kann bspw. mit einer Querschnittsdimension bereitgestellt werden, die im Bereich von 0.05 mm bis 10 cm gewählt ist.

Wenn das Kulturgefäß ein ausreichendes Volumen aufweist, können mehrere gasförmige Kultivierungsphasen voneinander getrennt in einer gemeinsamen flüssigen Kultivierungsphase angeordnet sein, um mehrere Grenzflächen zur Zellkultivierung bereitzustellen (keine Ausführungsform der Erfindung). Dies kann für die Kultivierung größerer Zellgruppen oder für den Vergleich der Kultivierungsergebnisse verschiedener Zellen von Vorteil sein.

Gemäß der Erfindung werden die Zellen an einer Grenzfläche zwischen zwei flüssigen Kultivierungsphasen angeordnet. Die Substanzen der aneinandergrenzenden Kultivierungsphasen sind so gewählt, dass die Kultivierungsphasen nicht miteinander mischbar sind. Alternativ kann zwischen den Kultivierungsphasen eine flexible Membran, wie z. B. eine biologische Doppelschicht vorgesehen sein.

Flüssige Kultivierungsphasen können schichtförmig übereinander eingestellt werden, um ebene Grenzflächen zu erzeugen (keine Ausführungsform der Erfindung). Gemäß der Erfindung sind die flüssigen Kultivierungsphasen so angeordnet, dass eine mindestens innere, flüssige Kultivierungsphase von einer äußeren, flüssigen Kultivierungsphase vollständig eingeschlossen wird.

Gemäß einer weiteren bevorzugten Ausführungsform der Erfindung ist vorgesehen, dass während oder am Ende der Zellkultivierung in mindestens einer der Kultivierungsphasen ein physikalischer oder chemischer Phasenübergang induziert wird. Es können bspw. unter sterilen Bedingungen Phasenübergänge in der Viskosität oder zwischen festen und flüssigen Zuständen induziert werden. Hierzu werden als Substanzen der Kultivierungsphasen vorzugsweise Polymere verwendet, deren Eigenschaften sich in Abhängigkeit von der Temperatur, z. B. im Bereich von 5 °C bis 40 °C, in Abhängigkeit vom pH-Wert oder in Abhängigkeit von der Ionenkonzentration stark ändern.

Derartige Polymer lassen sich durch eine Einstellung der Temperatur, der Ionenstärke und/oder des pH-Wertes von einer ersten in eine zweite Konformation schalten. Typischerweise werden in den verschiedenen Zuständen hydrophobe oder hydrophile molekulare Gruppen exponiert oder abgeschirmt. Dies ist zum Beispiel bei Polymeren der Fall, die zwischen einem kollabierten und einem ausgedehnten Zustand wechseln können. Beispiele für derartige Polymere sind Poly(N-Iso-propylacrylamid)(PNiPAA) oder Poly(N,N-Diethylacyl-amid)(PDEAAm) (siehe C. L. McCormick (ed.) in "Stimuliresponsive water-soluble and amphiphilic polymers", ACS Symposium, Series No. 780, American Chemical Society, Washington DC, 1999). Vorteilhafterweise können derartige Polymere zusammen mit funktionalen Polymeren (z. B. Polyethylenglykol, PEG) als Copolymere synthetisiert werden (siehe D. Schmaljojann in "Langmuir", Bd. 4, 2003, S. 1733). Entsprechend können in den Kultivierungsphasen verschiedene chemische und/oder physikalische Kultivierungsbedingungen eingestellt werden.

Gemäß einer weiteren Modifizierung kann erfindungsgemäß vorgesehen sein, dass mindestens zwei der Kultivierungsphasen miteinander verschmolzen werden. Zur Verschmelzung der Kultivierungsphasen werden die geometrischen, physikalischen oder chemischen Eigenschaften der beteiligten Kultivierungsphasen angeglichen, bis die Grenzfläche aufgehoben wird und die Kultivierungsphasen sich miteinander verbinden. Vorteilhafterweise können durch die Verschmelzung von Kultivierungsphasen Zellen oder Zellgruppen, die zunächst getrennt kultiviert wurden, miteinander in Kontakt gebracht werden. Beim Wachstum von Zellkulturen und/oder der Differenzierung von Zellen können sich Zellen gegenseitig beeinflussen, wie dies in der Natur z. B. bei der Embryogenese der Fall ist.

Alternativ kann eine Aufteilung von einer Kultivierungsphase in getrennte Kultivierungsphasen vorgesehen sein. Diese Ausführungsform der Erfindung ermöglicht vorteilhafterweise, dass eine zunächst einheitlich kultivierte Zellkultur in mindestens zwei Teile als Ausgangspunkt für eine weitere Kultivierung aufgeteilt werden kann.

Gemäß einer weiteren, bevorzugten Ausführungsform der Erfindung kann die Kultivierung der biologischen Zellen an der Grenzfläche zwischen nicht-festen Kultivierungsphasen durch die Bereitstellung eines festen Substratkörpers beeinflusst werden. Als Substratkörper ist vorzugsweise ein Gelkörper, ein Netz, ein Filament, ein textiles Substrat, eine Folie oder eine Zusammensetzung aus mindestens zwei dieser Substratkörpertypen vorgesehen. Vorzugsweise zeichnet sich der Substratkörper durch eine Biegsamkeit aus. Der Substratkörper kann an die Form der Grenzfläche zwischen Kultivierungsphasen angepasst werden. Gemäß einer weiteren vorteilhaften Variante weist der Substratkörper eine geringere Dichte als mindestens eine der an die Grenzfläche angrenzenden Kultivierungsphasen auf. In diesem Fall kann der Substratkörper auf der Grenzfläche schwimmen und/oder den Zellen ein Substrat bieten oder zur Kompartimentierung (Unterteilung) von Kultivierungsbereichen dienen. Der Substratkörper kann eine durchbrochene Oberfläche aufweisen und zum Beispiel eine Lochrasterfolie umfassen. Die an der Grenzfläche kultivierten Zellen können mit dem Substratkörper einen Verbund eingehen. Diese Ausführungsform der Erfindung ist besonders für die gezielte Erzeugung von Zellgewebe mit einer bestimmten Form und Zusammensetzung (tissue engineering) von Vorteil. Zur Bildung eines biokompatiblen Implantats kann der Substratkörper die Form des gewünschten Implantats aufweisen und mit den gewünschten Zellen besiedelt werden. Vorzugsweise ragt der Substratkörper von einer Kultivierungsphase in die benachbarte Kultivierungsphase. Die Oberfläche des Substrats ist von der Grenzfläche zwischen den Kultivierungsphasen abweichend ausgerichtet. In diesem Fall ist vorzugsweise in einer der Kultivierungsphasen die Zellvermehrung und -differenzierung vorgesehen, während in der anderen der Kultivierungsphasen das Wachstum der Zellen auf oder im Substratkörper induziert wird.

Ein weiterer wichtiger Vorteil der Erfindung besteht in der Vereinfachung der Manipulation biologischer Zellen. Erfindungsgemäß kann eine Zellentnahme, eine Zellgruppierung und/oder eine Zellzufuhr im Kulturgefäß vorgesehen sein. Vorteilhafterweise kann die selektive Entnahme oder Eingabe von Zellen wegen der Kultivierung an der Grenzfläche zwischen nicht-festen Kultivierungsphasen besonders schonend und sanft erfolgen. Einzelzellen, Zellgruppen oder Gewebe können enzymfrei und mechanisch stressarm aus den Kultivierungsphasen oder von den Grenzflächen entfernt oder umgekehrt zu diesen zugeführt werden. Vorteilhafterweise können in definierter Weise Mischkulturen aus verschiedenen Zelltypen, wie sie an die natürlichen Verhältnissen bei der Gewebebildung angepasst sind, in definierten und reproduzierbarer Weise hergestellt werden. Die Differenzierung und Ausformung von Zellaggregaten oder Gewebe kann gezielt durch die Bereitstellung bestimmter Nachbarschaften von Zelltypen oder Gewebeteilen physiologisch oder verträglich induziert werden.

Gemäß einer vorteilhaften Ausführungsform der Erfindung kann die Entnahme von Zellen oder Zellgruppen aus der Kultivierungseinrichtung in einem Zustand erfolgen, in dem die Zellen oder Zellgruppen mit dem Substratkörper verbunden sind. Der Substratkörper kann mit den auf diesem wachsenden Zellen oder Zellgruppen oder Gewebe aus den Kultivierungsphasen entnommen werden.

Vorzugsweise werden die Kultivierungsphasen in einer Kultivierungseinrichtung mit einem Kulturgefäß eingestellt, das an einer Oberseite offen ist oder eine verschließbare Öffnung aufweist. Das Kulturgefäß hat vorzugsweise eine formstabile Wand, mit der eine Aufnahme für die Kultivierungsphasen gebildet wird. Gemäß einer besonders bevorzugten Variante der Erfindung ist die Wand des Kulturgefäßes aus einem nichtnatürlichen, künstlichen oder synthetischen Material, wie z. B. Kunststoff, Glas oder Keramik, Halbleiter oder Metall hergestellt. Im Unterschied zu den herkömmlichen Techniken wird die Gestalt der mindestens einen Grenzfläche zwischen Kultivierungsphasen nicht durch die Innenform des Kulturgefäßes, sondern durch die Kultivierungsphase-Einstellung gegeben.

Weitere Einzelheiten und Vorteile der Erfindung werden im Folgenden unter Bezug auf die beigefügten Zeichnungen beschrieben. Es zeigen:
- Figuren 1 bis 28:: Illustrationen der Einstellung von Kultivierungsphasen zur Kultivierung biologischer Zellen, wobei die Figuren 3, 4, 7, 8 und 10 bis 24 Ausführungsformen der Erfindung und die Figuren 1, 2, 5, 6, 9 und 25 bis 28 keine Ausführungsformen der Erfindung darstellen;
- Figur 29:: eine zur Durchführung des erfindungsgemäßen Verfahrens verwendbare Kultivierungseinrichtung;
- Figur 30:: eine weitere Kultivierungseinrichtung; und
- Figur 31:: eine Illustration der herkömmlichen Zellkultivierung in hängenden Tropfen.

Die in den Figuren beispielhaft illustrierten Kultivierungsverfahren können mit allen biologischen Zellen durchgeführt werden, die sich auch mit den herkömmlichen Kultivierungstechniken vermehren und/oder differenzieren lassen. Es können bspw. embryonale oder adulte Stammzellen verschiedenen Ursprungs aus humanen oder tierischen Organismen oder differenzierte Zellen aus humanen oder nicht-humanen, insbesondere tierischen Organismen kultiviert werden. Die illustrierten Verfahren wurden z. B. mit genetisch modifizierten Zellen getestet, die z. B. Zellen mit einer Expression von Markergenen, wie z. B. LacZ oder GFP, Zellen mit einem Gen-"Knockout", Zellen mit einem Gen-"Silencing" oder Zellen mit einer Gen-Überexpression umfassen.

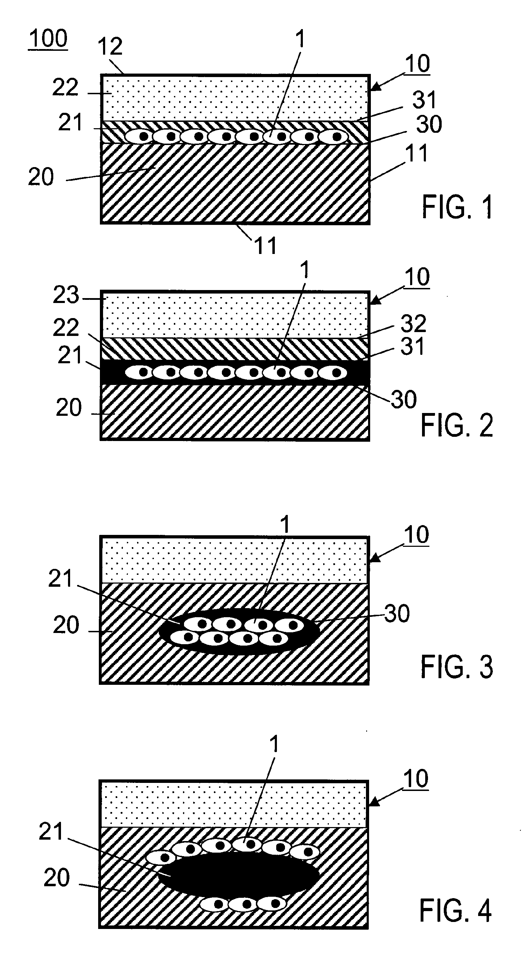

Bei den Illustrationen in den Figuren 1 bis 28 umfasst die zur Verfahrensführung verwendete Kultivierungseinrichtung 100 ein Kulturgefäß 10, in dem mehrere Kultivierungsphasen 20, 21, 22, ... angeordnet sind. Obwohl in allen Fällen eine gasförmige Kultivierungsphase gezeigt ist, muss diese nicht zwingend vorgesehen sein. Gemäß der Erfindung werden im Kulturgefäß flüssige Kultivierungsphase eingestellt. Das Kulturgefäß 10 (schematische Schnittdarstellungen) umfasst ein Behältnis, z. B. mit einer Quaderform, einer Zylinderform oder einer anderen Volumenform, das geschlossene Boden- und Seitenwände 11 (siehe Figur 1) und an der Oberseite eine Öffnung 12 aufweist. Die Öffnung 12 kann ggf. mit einem Deckel (nicht dargestellt) verschlossen werden. Das Kulturgefäß 10 umfasst bspw. eine Kunststoffbox, -flasche oder -schale oder ein Becherglas. Das Volumen des Kulturgefäßes 10 wird in Abhängigkeit von der konkreten Kultivierungsaufgabe im Bereich von z. B. 0.2 ml bis 10 l gewählt. Die Kultivierungsphasen 20, 21, 22, ... im Kulturgefäß 10 können erfindungsgemäß entsprechend den im Folgenden erläuterten Varianten oder Kombinationen aus diesen eingestellt werden.

Gemäß Figur 1 sind im Kulturgefäß 10 zwei flüssige Kultivierungsphasen 20, 21 und eine gasförmige Kultivierungsphase 22 schichtförmig angeordnet. Zwischen den Kultivierungsphasen sind ebene Grenzflächen 30, 31 gebildet (keine Ausführungsform der Erfindung). Die flüssigen Kultivierungsphasen 20, 21 umfassen nicht-mischbare flüssige Medien, die z. B. aus einer der folgenden Kombinationen gewählt sind. Typischerweise umfasst die untere flüssige Kultivierungsphase 20 eine Trägerflüssigkeit, wie z. B. Silikonöl, eine Polymerlösung oder ein Gel. Die mittlere flüssige Kultivierungsphase 21 umfasst vorzugsweise ein Nährmedium für biologische Zellen. Die Massendichte der unteren Kultivierungsphase ist höher als die Massendichte der mittleren Kultivierungsphase und der Zellen. Die obere gasförmige Kultivierungsphase 22 wird durch einen Volumenbereich im Kulturgefäß 10 gebildet, der mit einem Versorgungsgas oder -dampf, wie z. B. CO₂, O₂ oder dgl. gefüllt ist.

Die Einstellung der Kultivierungsphasen 20, 21 erfolgt derart, dass zunächst die schwerere Kultivierungsphase 20 in das Kulturgefäß 10 eingefüllt und anschließend mit der leichteren Kultivierungsphase 21 überschichtet wird. Die biologischen Zellen 1 werden mit der zweiten Kultivierungsphase 21 oder bei einem anschließenden Injektionsschritt mit einer Zellsuspension in das Kulturgefäß 10 eingeführt. Die Zellen 1 sinken in der zweiten Kultivierungsphase 21 ab, bis sie an der Grenzfläche 30 auf der ersten Kultivierungsphase 20 schwimmen.

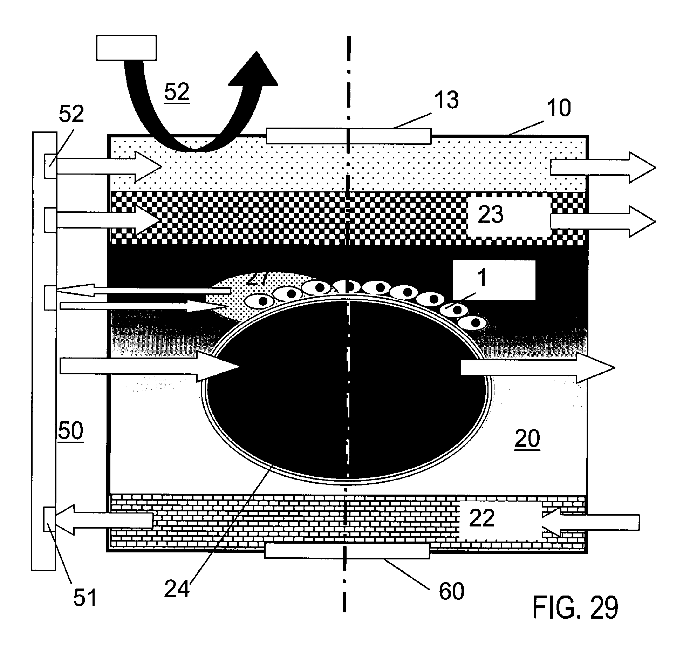

Nach der Einstellung der Kultivierungsphasen 20, 21, 22 mit der Ablage der Zellen 1 an der Grenzfläche 30 werden ggf. weitere Kultivierungsbedingungen, wie z. B. die Temperatur eingestellt. Hierzu wird eine Temperierungseinrichtung (siehe Beschreibung von Figur 29) verwendet, mit der die Temperatur des Gefäßes einstellbar ist.

Gemäß Figur 2 sind drei flüssige Kultivierungsphasen 20, 21, 22 und eine gasförmige Kultivierungsphase 23 vorgesehen, die schichtförmig mit ebenen Grenzflächen 30, 31, 32 im Kulturgefäß 10 angeordnet sind (keine Ausführungsform der Erfindung). Die Zellen 1 befinden sich in der mittleren Kultivierungsphase 21, die z. B. ein Nährmedium für die Zellen umfasst und beidseitig durch die untere und obere Kultivierungsphase 20, 22 begrenzt wird. Die mittlere Kultivierungsphase 21 bildet einen schichtförmigen, nahezu zweidimensionalen Volumenbereich zur Bildung einer Monolage der Zellen 1 z. B. mit einer Dicke von bis zu 5 mm. Es können alternativ größere Dicken zur Herstellung von mehrschichtigen Zellkulturen vorgesehen sein.

In den Figuren 3 und 4 sind Ausführungsformen der Erfindung mit zwei flüssige Kultivierungsphasen 20, 21 mit einer gekrümmten Grenzfläche 30 gezeigt. Die Zellen 1 sind auf der Innenseite (Figur 3) oder Außenseite (Figur 4) der Grenzfläche 30 angeordnet. Die Substanzen der Kultivierungsphasen 20, 21 sind zwei fließfähige, insbesondere flüssige Zusammensetzungen, die nicht mischbar sind, jedoch vorzugsweise eine ähnliche Massendichte aufweisen. Beispielsweise besteht gemäß Figur 3 die innere Flüssigkeitsphase 21 aus einem Nährmedium, das außen allseits von einer zähflüssigen Polymerlösung umgeben wird. Alternativ können das Nährmedium außen und die Polymerlösung innen vorgesehen sein. Es ist nicht zwingend erforderlich, dass die Zellen 1 in dem Nährmedium angeordnet sind. Wenn die Zellen an der Grenzfläche 30 z. B. auf der Seite der Polymerlösung kultiviert werden, können Nährstoffe vom Nährmedium durch die Grenzfläche zu den Zellen diffundieren.

Die in den Figuren 3 und 4 gezeigte gekrümmte, geschlossene Form der Grenzfläche 30 stellt vorteilhafterweise geometrische Kultivierungsbedingungen bereit, die denen in einem natürlichen Wirts-Ei oder in hängenden Tropfen ähnlich sind. Im Unterschied zu den herkömmlichen Techniken werden die Größe und die Form der Grenzfläche 30 in Abhängigkeit von der konkreten Kultivierungsaufgabe gezielt eingestellt.

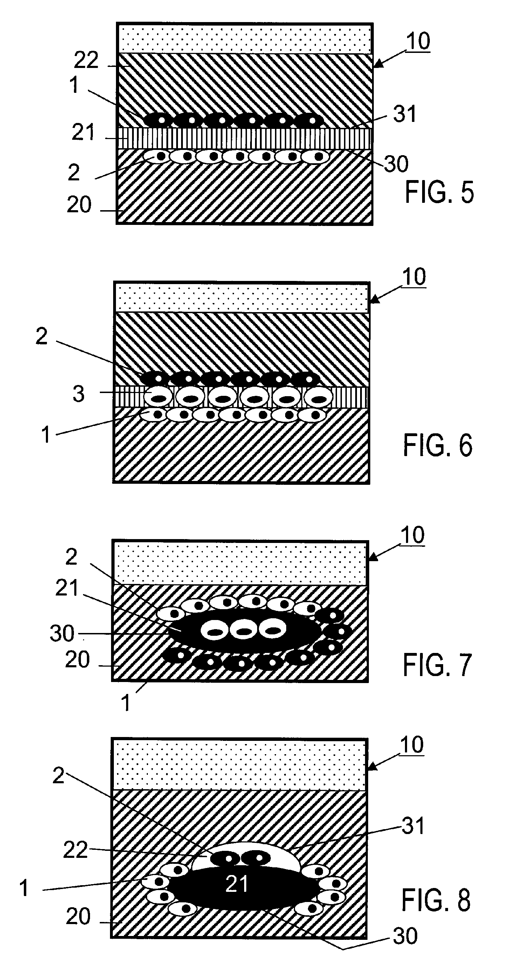

Die Figuren 5 bis 8 illustrieren die Kultivierung von Zellen 1, 2 mit verschiedenen Zelltypen in verschieden eingestellten Kultivierungsphasen analog zu den Figuren 1 bis 4. Gemäß Figur 5 sind drei flüssige Kultivierungsphasen 20, 21 und 22 im Kulturgefäß 10 schichtförmig angeordnet (keine Ausführungsform der Erfindung). Die Zellen 1, 2 der verschiedenen Zelltypen werden an den Grenzflächen 30, 31 in den räumlich voneinander getrennten Kultivierungsphasen 20, 22 kultiviert. Dabei wachsen und differenzieren sich die Zellen räumlich getrennt. Durch die mittlere Kultivierungsphase 21 können jedoch Signalstoffe zwischen den Zellen 1, 2 durch Diffusion ausgetauscht werden, bspw. um die Differenzierung der Zellen gegenseitig zu beeinflussen. Hierzu weist die mittlere Kultivierungsphase 21 eine Dicke vorzugsweise im Bereich von 10 µm bis 10 mm auf.

Figur 6 illustriert den entsprechenden Fall mit einer Kultivierung von Zellen 1, 2 und 3 von drei verschiedenen Zelltypen (keine Ausführungsform der Erfindung). Die Zellen 3 des dritten Zelltyps werden in der mittleren Kultivierungsphase kultiviert. Die verschiedenen Zelltypen umfassen z. B. als Zellen 1 Fibroblasten, die als Feeder-Schicht wirken, Zellen 2, die Muskelzellen oder ebenfalls Fibroblasten umfassen, und Zellen 3, die Stammzellen oder Vorläuferzellen umfassen, die bei der Kultivierung differenziert werden sollen.

Die Figuren 7 und 8 zeigen die Kultivierung verschiedener Zelltypen an gekrümmten Grenzflächen 30, 31. Gemäß Figur 7 ist gemäß einer Ausführungsform der Erfindung analog zu Figur 4 eine nicht-mischbare Kultivierungsphase 21 als Flüssigkeitsellipsoid in die äußere Kultivierungsphase 20 eingefügt. Die innere Kultivierungsphase 20 kann eine höhere Viskosität als die äußere Kultivierungsphase 21 aufweisen oder z. B. Protein- und/oder Nährstoffmischungen enthalten. An der Grenzfläche 30 zwischen den Kultivierungsphasen 21, 22 sind Zellen 1, 2 verschiedener Zelltypen angeordnet, während Zellen 3 eines dritten Zelltyps in der inneren Kultivierungsphase 21 wachsen und sich differenzieren können.

Bei der Variante gemäß Figur 8 sind gemäß einer Ausführungsform der Erfindung von der äußeren Flüssigkeitsphase 22 zwei innere Flüssigkeitsphasen 21, 22 umschlossen, wobei gekrümmte Grenzflächen 30, 31 gebildet werden. Durch diese Einstellung der Kultivierungsphasen können die Zellen 1, 2 verschiedener Zelltypen räumlich getrennt, jedoch im stofflichen Diffusionsaustausch in gegenseitiger Wechselwirkung kultiviert werden.

Entsprechend den in den Figuren 5 bis 8 gezeigten Beispielen können kompliziertere Strukturen von Flüssigkeitsphasen eingestellt werden, um Zellen einzelner oder mehrerer Zelltypen zu kultivieren. Vorteilhafterweise können damit komplexe Zellanordnungen konstruiert werden, deren geometrische Verteilung entsprechend der Verteilung differenzierter Zellen in den Keimblättern im Verlauf der Embryogenese gewählt und stabil gehalten wird. Analog können auch aus verschiedenen, fertig differenzierten Zellen, die im Rahmen der erfindungsgemäßen Kultivierung vermehrt werden, komplexe Gewebestrukturen erzeugen.

Die Einstellung der Kultivierungsphasen 20, 21, 22 erfolgt derart, dass zunächst die äußere Kultivierungsphase 20 in das Kulturgefäß 10 eingefüllt und anschließend mit die inneren Kultivierungsphase(n) 21 (, 22) injiziert werden. Die biologischen Zellen 1 werden mit der inneren Kultivierungsphase oder bei einem anschließenden Injektionsschritt mit einer Zellsuspension in das Kulturgefäß 10 eingeführt.

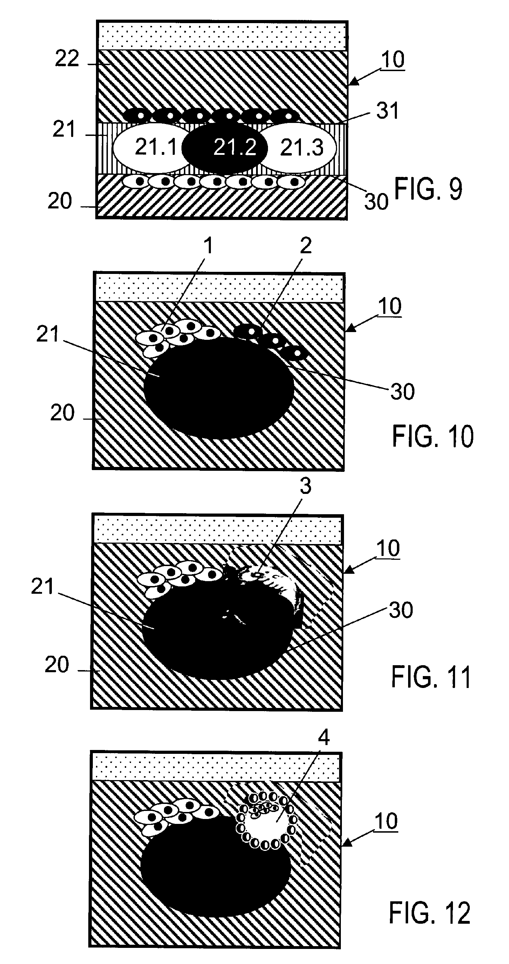

Figur 9 illustriert eine Kultivierungseinrichtung 100 mit drei flüssigen Kultivierungsphasen 20, 21 und 22, die wie in Figur 5 schichtförmig mit ebenen Grenzflächen 30, 31 angeordnet sind (keine Ausführungsform der Erfindung). In die mittlere Flüssigkeitsphase 21 sind Zusatzstoffe 21.1, 21.2 und 21.3 eingeführt, die z. B. verschiedene Wachstumsfaktoren umfassen. Die Wachstumsfaktoren gelangen durch Diffusion über die Grenzflächen 30, 31 zu den Zellen 1, 2. Die Zusatzstoffe 21.1, 21.2 und 21.3 können bspw. als Festkörperpartikel in die mittlere Flüssigkeitsphase 21 eingeführt werden. Sie bestehen z. B. aus Keramik, Gelmatrizen, Gewebe- oder Textilelementen oder mikro- bzw. nanoporösen Partikeln unterschiedlicher oder gleicher Größe.

Die Figuren 10 und 11 illustrieren die Entwicklung komplexer Zellstrukturen an der Grenzfläche 30 zwischen zwei flüssigen Kultivierungsphasen 20, 21 analog zu Figur 4 (Ausführungsformen der Erfindung). Gemäß Figur 10 werden auf der Grenzfläche 30 zueinander benachbart zwei Zellhaufen kultiviert, die sich in ihrem Wachstums- und Differenzierungsverhalten beeinflussen. Die Zellen 1, 2 umfassen bspw. adulte Stammzellen, embryonale Stammzellen, Feeder-Zellen, embryonale differenzierte Zellen, adulte differenzierte Zellen, Tumorzellen, Eizellen, Vorläuferzellen, dendritische Zellen, Fibroplastenzellen und/oder Zellen der tierischen Keimscheibe. Die Zellen können einen losen Zellverbund oder ein Gewebestück bilden.

Gemäß Figur 12 werden tierische Zellen in Nachbarschaft zu einem Embryo oder Fötus 3 tierischer Herkunft (z. B. Huhnembryo) kultiviert (Ausführungsform der Erfindung). Der Embryo kann gemäß Figur 12 durch eine Einzelle (Oozyte) oder Zygote oder einen Zellhaufen in einem höheren Teilungsstadium bis zur Blastozyste 4 ersetzt werden.

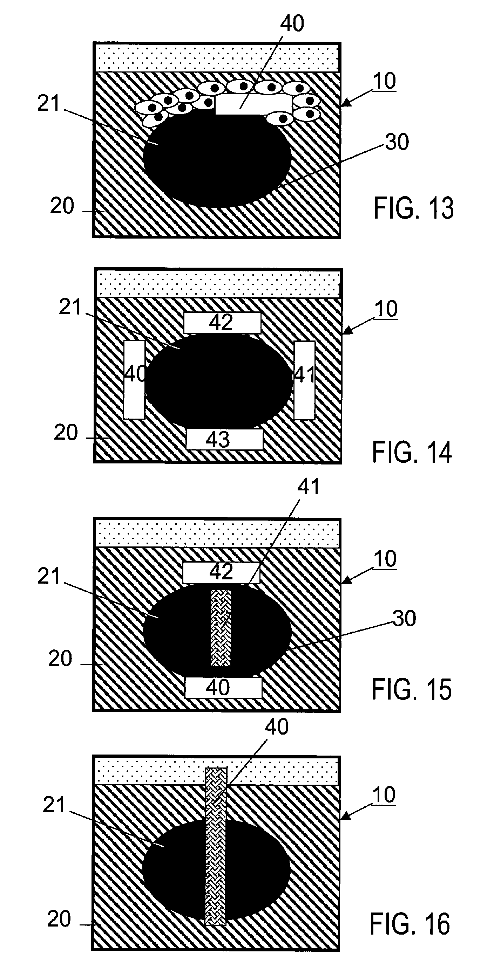

Die Figuren 13 bis 16 illustrieren verschiedene Varianten der Zellkultivierung in Gegenwart eines festen Substratkörpers 40, 41, ..., der von den an einer Grenzfläche 30 kultivierten Zellen partiell oder vollständig be- oder durchwachsen werden soll (Ausführungsformen der Erfindung). Der Substratkörper kann ein sog. Scaffold (Trägerstruktur) z. B. aus Keramik, Titan, Kunststoff oder anderen, in der Implantationsmedizin üblichen Materialien sein. Der Substratkörper 40, 41 ..., besteht z. B. aus einem textilen Gewebe, einer Kunststoffmatrix, einer Keramik, Metall, Glas oder einem Halbleiter. In den flüssigen Kultivierungsphasen 20, 21 kann z. B. ein Knochenimplantat, Knorpelimplantat oder Herzklappenimplantat als Substratkörper 40, 41, ... angeordnet werden. Gemäß Figur 14 können mehrere Substratkörper 40 - 43 in vorbestimmten Relativpositionen angeordnet werden, um von den Zellen (nicht dargestellt), die an der Grenzfläche 30 zwischen den Kultivierungsphasen 20, 21 kultiviert werden, umwachsen zu werden.

Gemäß Figur 15 ist einer der Substratkörper 41 ausschließlich in der inneren Flüssigkeitsphase 21 angeordnet. Mit der Kultivierungseinrichtung gemäß Figur 15 können bspw. auf der Innenseite der Grenzfläche 30 tierische Inselzellen kultiviert werden, die auf den inneren Substratkörper 41 aufwachsen, während auf der Außenseite der Grenzfläche 30 Zellen eines Patienten kultiviert werden, die mit den äußeren Substratkörpern 40, 42 verbunden werden. Im Ergebnis der Zellkultivierung wird ein komplexes Gebilde geschaffen, bei dem die tierischen Inselzellen von der Umgebung durch die humanen Zellen des behandelten Patienten ummantelt und von der Umgebung getrennt sind. Die Substratkörper 40, 41 und 42 stabilisieren vorteilhafterweise diese Zellstruktur.

Gemäß Figur 16 kann der Substratkörper 40 verwendet werden, um getrennte Kompartimente im Kulturgefäß 10 zu schaffen. In den getrennten Kompartimenten können Zellen verschiedener Zelltypen (nicht dargestellt) kultiviert werden. Der Substratkörper 40 kann eine poröse Struktur aufweisen und für flüssige oder gasförmige Substanzen permeabel oder semipermeabel sein. Des Weiteren kann ein Substratkörper, wie dies in Figur 16 beispielhaft illustriert ist, der Gaszufuhr aus der gasförmigen Kultivierungsphase 22 in die flüssigen Kultivierungsphasen 20, 21 dienen. Der poröse Substratkörper 40 besteht z. B. aus einer porösen Keramik oder einem Kunststoffschaum.

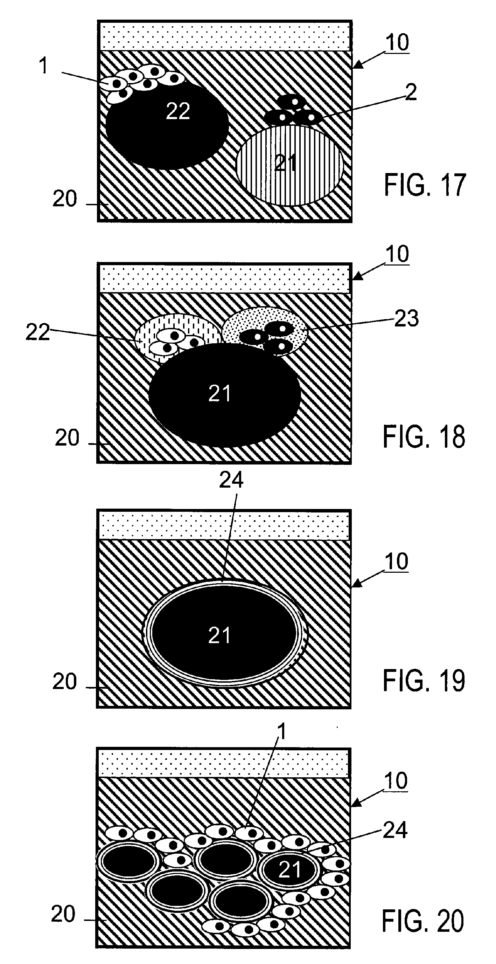

Die Figuren 17 und 18 illustrieren weitere Beispiele für Mehrphasensysteme mit zwei oder drei inneren Kultivierungsphasen 21, 22, ... die allseits von einer äußeren Kultivierungsphase 20 umschlossen sind (Ausführungsformen der Erfindung). Gemäß Figur 17 werden auf den getrennten Kultivierungsphasen 21, 22, ... Zellen 1, 2 verschiedener Zelltypen kultiviert. Mit der Einstellung getrennter Kultivierungsphasen 21, 22 wird vorteilhafterweise eine Trennung der Zelltypen erreicht. Vorteilhafterweise werden die Zellen 1, 2 der verschiedenen Zelltypen getrennt kultiviert, wobei jedoch eine chemische Verbindung mittels Diffusion durch die Flüssigkeitsphase 20 gegeben ist. Die Substanzen der Flüssigkeitsphasen 21, 22 können in Abhängigkeit von der konkreten Kultivierungsaufgabe verschieden gewählt sein und z. B. Nährmedien, Hormone, Signalfaktoren, Differenzierungsfaktoren, Wachstumsfaktoren etc. enthalten. Die Kultivierungseinrichtung gemäß Figur 17 kann somit als Abwandlung herkömmlicher Feeder-Kulturen zur Stabilisierung von embryonalen Stammzellen oder zur Differenzierung von anderen Stammzellen verwendet werden.

Figur 18 zeigt analog zu Figur 8 ein Drei-Phasen-System, bei dem die erste innere Kultivierungsphase 21 als Träger für die anderen inneren Kultivierungsphasen 22, 23 in der äußeren Kultivierungsphase 20 dient. In den Kultivierungsphasen 22, 23 können analog zu Figur 17 verschiedene Substanzen enthalten sein.

Figur 19 zeigt eine Variante der Erfindung, bei der an der Grenzfläche zwischen den flüssigen Kultivierungsphasen 20, 21 eine Membranschicht 24 vorgesehen ist (Ausführungsform der Erfindung). Die Membranschicht 24 umhüllt die innere Kultivierungsphase 21. Die zu kultivierenden Zellen (nicht dargestellt) liegen innen und/oder außen auf der Membranschicht 24 auf. Die Membranschicht umfasst eine dünne Schicht, z. B. aus Lipiden und/oder Proteinen mit einer Dicke im Sub-mm-Bereich. Mit der Membranschicht 24 kann die Anwendung der Erfindung vorteilhafterweise auf Kultivierungsphasen mit Substanzen ausgedehnt werden, die miteinander mischbar sind. Die Membranschicht 24 entspricht den in natürlichen Verhältnissen bei der Zellentwicklung im Embryo und im Organismus gegebenen Trennschicht zwischen verschieden differenzierten Zellbereichen.

Gemäß Figur 20 können in der äußeren flüssigen Kultivierungsphase 20 mehrere innere flüssige Kultivierungsphase 21 angeordnet sein, die jeweils von einer Membranschicht 24 umhüllt sind (Ausführungsform der Erfindung). Auf der Oberfläche der Gruppe von membranumhüllten Kultivierungsphasen 21 können die biologischen Zellen 1 wachsen, sich vermehren und differenzieren. Abweichend von der schematischen Illustration können die Membran umhüllten Kultivierungsphasen bis zu mikroskopisch kleinen Tropfen, wie z. B. Vesikeln oder Liposomen verkleinert werden, die kleiner oder gleich den zu kultivierenden Zellen sind.

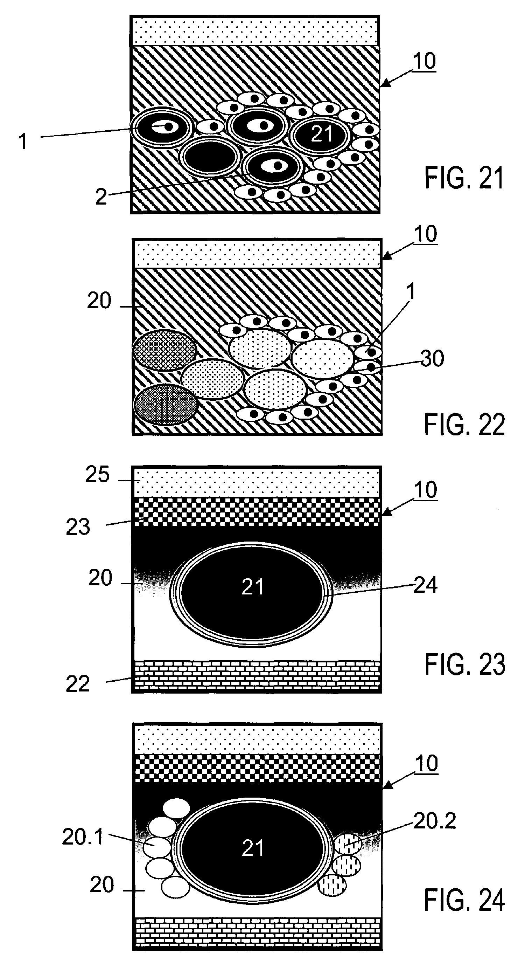

Gemäß Figur 21 können in einzelnen oder allen membranumhüllten Kultivierungsphasen 21 Zellen 1, 2 angeordnet sein. Die Zellen 1, 2 können von gleichen oder verschiedenen Zelltypen sein (Ausführungsform der Erfindung). Die Kultivierungseinrichtung gemäß Figur 21 stellt eine sog. Ammenkultur dar.

Die inneren flüssigen Kultivierungsphasen 21 können gleichförmig aus bestimmten Substanzen (siehe z. B. Figur 17) bestehen oder alternativ innerhalb der äußeren, flüssigen Kultivierungsphase 20 einen Substanzgradienten bilden, wie dies schematisch in Figur 22 illustriert ist (Ausführungsform der Erfindung). Die inneren Kultivierungsphasen 21 enthalten z. B. Nährmedien, Ionen, Wachstumsfaktoren, Signalfaktoren oder dgl. mit verschiedenen Konzentrationen, so dass ein Gradient entsteht. Entsprechend dem Verlauf des Gradienten sind Zellen 1 auf der äußeren Seite der Grenzflächen 30 zur äußeren Kultivierungsphase 20 verschiedenen Konzentrationen der entsprechenden Substanzen ausgesetzt. In der erfindungsgemäßen Kultivierungseinrichtung können in Abhängigkeit von der konkreten Kultivierungsaufgabe verschiedene Gradienten physikalischer oder chemischer Kulturbedingungen eingestellt werden. Da die inneren Kultivierungsphasen 21 miniaturisiert werden können, lassen sich die Gradienten zwei- oder dreidimensional und ggf. stufenlos einstellen. Die inneren Kultivierungsphasen 21 können z. B. einen planaren Vesikelteppich mit einem Substanzgradienten bilden.

Die Gradientenbildung kann auch in der äußeren Kultivierungsphase 20 vorgesehen sein, wie dies schematisch in den Figuren 23 und 24 illustriert ist (Ausführungsformen der Erfindung). Es sind vier flüssige Kultivierungsphasen 20 bis 23 und eine gasförmige Kultivierungsphase 25 vorgesehen. Die Kultivierung der Zellen (nicht dargestellt) erfolgt auf der Innen- und/oder Außenseite der Membranschicht 24 zwischen der äußeren Kultivierungsphase 20 und der inneren Kultivierungsphase 21. Die zusätzlichen Kultivierungsphasen 22 und 23 sind im Kulturgefäß 10 schichtförmig oder- und unterhalb der äußeren Kultivierungsphase 20 angeordnet und dienen der Erzeugung eines schematisch illustrierten Gradienten in der äußeren Kultivierungsphase 20. Figur 24 zeigt die entsprechende Einstellung der Kultivierungsphasen, wobei zusätzlich Nährstoffdepots 20.1, 20.2 in der äußeren Kultivierungsphase 20 vorgesehen sind. Die zusätzlichen Nährstoffdepots 20.1, 20.2 dienen als weitere Diffusionsquellen zur Beeinflussung der chemischen Kulturbedingungen in der Kultivierungseinrichtung.

Erfindungsgemäß wird vorzugsweise mindestens ein chemischer Gradient erzeugt, der vertikal ausgerichtet ist. Es kann vorgesehen sein, dass sich der vertikale Gradient im Zeitverlauf langsam auflöst oder sich durch die Kultivierungsphasen bewegt. Die Bewegung kann z. B. durch Auftrieb oder Sedimentation erfolgen.

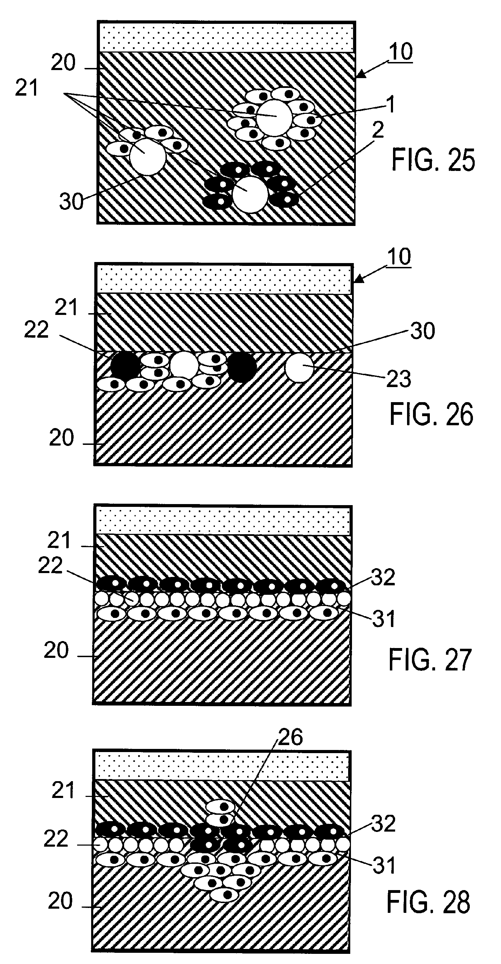

Die Figuren 25 bis 28 illustrieren eine Kultivierungseinrichtung, in der Kulturbedingungen ähnlich zur Zellkultivierung in hängenden Tropfen bereitgestellt werden, wobei die geometrischen Eigenschaften von Grenzflächen 30 zwischen einer flüssigen Kultivierungsphase 20 und mindestens einer gasförmigen Kultivierungsphase 21 durch die erfindungsgemäße Kultivierungsphasen-Einstellung wählbar sind. Gemäß Figur 25 werden im Kulturgefäß 10 in die flüssige Kultivierungsphase 20 Gasblasen 21 als gasförmige Kultivierungsphasen eingefügt (keine Ausführungsform der Erfindung). Es wurde festgestellt, dass mit dem gasförmig-flüssig-System gemäß Figur 25 eine ausreichende Stabilität zur Kultivierung erreicht werden kann. Hierzu wird bspw. die Viskosität der flüssigen Kultivierungsphase 20 so hoch eingestellt, dass innerhalb der Kultivierungszeit die in der flüssigen Kultivierungsphase 20 angeordneten gasförmigen Kultivierungsphasen 21 nicht an die Oberfläche wandern können. Die Zellen 1, 2 werden an den Grenzflächen 30, insbesondere auf der Außenseite der gasförmigen Kultivierungsphasen 21 kultiviert.

Figur 26 zeigt eine entsprechende Struktur in einem Mehrphasensystem mit zwei schichtförmig angeordneten flüssigen Kultivierungsphasen 20, 21, an deren Grenzfläche 30 flüssige (22) und gasförmige (23) Kultivierungsphasen vorgesehen sind (keine Ausführungsform der Erfindung). Entsprechend wird eine komplexe Grenzfläche gebildet, an der die Zellkultivierung erfolgt. Vorteilhafterweise können mit der Kultivierungsphasen-Einstellung gemäß Figur 26 asymmetrische Zellkulturen erzeugt werden, wie sie für die Stammzelldifferenzierung erwünscht sind.

Gemäß einer weiteren Verfahrensvariante kann eine gasförmige Kultivierungsphase 22 in Form einer Vielzahl von Gasblasen zwischen zwei flüssigen Kultivierungsphasen 20, 21 angeordnet werden, wie dies schematisch in den Figuren 27 und 28 gezeigt ist (keine Ausführungsformen der Erfindung). Es werden Drei-Phasen-Systeme geschaffen, in denen zwei Grenzflächen 31, 32 zur Kultivierung von Zellen gleichen oder verschiedenen Typs bereitgestellt werden. Gemäß Figur 28 kann die Schicht der gasförmigen Kultivierungsphase 22 Unterbrechungen aufweisen, so dass Zellen durch eine Lücke 26 zwischen den beiden Seiten der Grenzflächen 31, 32 ausgetauscht werden und ineinander wachsen können.

Figur 29 zeigt eine Kultivierungseinrichtung 100 mit einem Kulturgefäß 10 und Kultivierungsphasen 20 mit weiteren Einzelheiten am Beispiel der Struktur gemäß Figur 23. Das Kulturgefäß 10 ist ein Behältnis für die flüssigen oder gasförmigen Kultivierungsphasen 20, in Form z. B. eines Becherglases. Es ist eine Injektionseinrichtung 50 (schematisch dargestellt) mit mindestens einem Flüssigkeitsinjektor 51 und mindestens einem Gasinjektor 52 zur Zufuhr von Zusatzstoffen in die einzelnen Kultivierungsphasen vorgesehen. Die Pfeile in Figur 29 illustrieren schematisch den Stoffaustausch von den Kultivierungsphasen mit der Umgebung. Der mindestens eine Gasinjektor 52 dient der Zufuhr von z. B. O₂ oder CO₂ und ggf. als Befeuchtungseinrichtung zur Einstellung einer bestimmten Umgebungsfeuchte in der gasförmigen Kultivierungsphase 26. Am Boden des Kulturgefäßes 10 ist eine schematisch illustrierte Temperierungseinrichtung 60 angeordnet. Die Oberseite des Kulturgefäßes 10 ist verschlossen. Es ist ein Beobachtungsfenster 13 vorgesehen, durch das die kultivierten Zellen beobachtet und ggf. beleuchtet werden können.

Mit dem mindestens einem Flüssigkeitsinjektor 51 kann die chemische Zusammensetzung jeder der flüssigen Kultivierungsphasen 20 eingestellt werden. Mit den schichtförmigen oberen und unteren Kultivierungsphasen kann analog zu Figur 23 ein Gradient in der mittleren Kultivierungsphase erzeugt werden. Die zu kultivierenden Zellen 1 wachsen auf der äußeren Oberfläche der Membranschicht 24, wobei ggf. zusätzlich eine Kultivierungsphase 27 mit einem Nährmedium vorgesehen ist.

Figur 30 illustriert ein weiteres Kulturgefäßes 10 in Form eines Zylinders zur Kultivierungsphasen-Einstellung gemäß der Erfindung. Im Kulturgefäß 10 sind schichtförmig eine untere (20) und eine obere (21) flüssige Kultivierungsphase vorgesehen, die eine mittlere (22) flüssige Kultivierungsphase einschließen (Figur 30A). Zur Einstellung der mittleren Kultivierungsphase 22 werden eine oder mehrere Injektionsleitungen 14 verwendet, mit denen die Substanz der mittleren flüssigen Kultivierungsphase 22 (Gas oder Flüssigkeit) und ggf. eine Zellsuspension in das Kulturgefäß 10 einführbar sind. Die anderen Kultivierungsphasen 20, 21 oder weitere Kultivierungsphasen können entsprechend durch Injektionsleitungen eingestellt werden. An der Oberseite des Kulturgefäßes 10 ist ein Verschluss 15 mit einem Gasdiffusionseinsatz 16 vorgesehen, durch den die gasförmige Kultivierungsphase 23 versorgt werden kann. Das Teilbild in Figur 30B zeigt die Draufsicht auf das Kulturgefäß 10 mit den Medienleitungen 14 und dem Verschluss 15.

## Patentansprüche

1. Verfahren zur Zellkultivierung von biologischen Zellen, ausgenommen humane embryonale Stammzellen, an mindestens einer gekrümmten Grenzfläche (30-32) zwischen mindestens zwei verschiedenen, nicht-festen Kultivierungsphasen (20-23) in einem Kulturgefäß (10) einer Kultivierungseinrichtung (100), mit den Schritten:
- Kultivierungsphasen-Einstellung, so dass vorbestimmte Kultivierungsbedingungen gegeben sind, wobei in der Kultivierungseinrichtung (100) zwei flüssige, nicht mischbare Kultivierungsphasen (20-23) eingestellt werden, wobei mindestens eine innere Kultivierungsphase von einer äußeren Kultivierungsphase vollständig eingeschlossen wird, und
- Kultivierung der biologischen Zellen an der mindestens einen Grenzfläche (30-32),
**dadurch gekennzeichnet, dass**
- die Kultivierungsphasen in dem Kulturgefäß (10) angeordnet werden, wobei zunächst die äußere Kultivierungsphase (20) in das Kulturgefäß (10) eingefüllt und anschließend die innere Kultivierungsphase (21) injiziert wird und die Kultivierungsphasen-Einstellung in dem Kulturgefäß (10) vor oder während einer Ablage der Zellen (1) an der mindestens einen Grenzfläche (30-32) erfolgt,
- die Kultivierungsphasen-Einstellung eine Beeinflussung der geometrischen Eigenschaften der Kultivierungsphasen (20-23) umfasst, so dass die mindestens eine Grenzfläche (30-32) zwischen den Kultivierungsphasen (20-23) eine vorbestimmte Form erhält, und eine Anordnung der Kultivierungsphasen (20-23) relativ zueinander umfasst, so dass die Grenzfläche (30-32) eine vorbestimmte Ausdehnung aufweist, wobei mit einer Injektionseinrichtung (50) in die Kultivierungsphasen (20-23) in der Kultivierungseinrichtung (100) Zusatzstoffe eingeführt werden, mit denen die Massendichte der Kultivierungsphasen (20-23) verändert und die mindestens eine Grenzfläche (30-32) in der Kultivierungseinrichtung (100) positioniert und in ihrer Form stabilisiert wird, und
- die Kultivierung eine Differenzierung der biologischen Zellen durch Signalfaktoren und/oder Differenzierungsfaktoren, die in mindestens einer der Kultivierungsphasen (20-23) enthalten sind, umfasst.

2. Verfahren nach Anspruch 1, bei dem die Kultivierungsphasen-Einstellung eine Bildung der Kultivierungsphasen (20-23) mit vorbestimmten Kultivierungsmedien umfasst.

3. Verfahren nach mindestens einem der vorhergehenden Ansprüche, bei dem die Kultivierungsphasen-Einstellung eine Bildung der Kultivierungsphasen (20-23) mit vorbestimmten Zusatzstoffen umfasst, mit denen wenigstens eines der Viskosität und der Mischbarkeit der Kultivierungsphasen beeinflusst wird.

4. Verfahren nach mindestens einem der vorhergehenden Ansprüche, mit dem Schritt:
- Zufuhr eines Gases zu den biologischen Zellen.

5. Verfahren nach mindestens einem der vorhergehenden Ansprüche, mit dem Schritt:
- Bereitstellung einer Membran (24) an der Grenzfläche (30-32) zwischen zwei Kultivierungsphasen (20-23).

6. Verfahren nach mindestens einem der vorhergehenden Ansprüche, mit dem Schritt:
- Erzeugung eines Phasenübergangs in mindestens einer der Kultivierungsphasen (20-23).

7. Verfahren nach mindestens einem der vorhergehenden Ansprüche, mit dem Schritt:
- Verschmelzung von mindestens zwei der Kultivierungsphasen (20-23).

8. Verfahren nach mindestens einem der vorhergehenden Ansprüche, mit dem Schritt:
- Aufteilung von mindestens einer der Kultivierungsphasen (20-23) in getrennte Kultivierungsphasen.

9. Verfahren nach mindestens einem der vorhergehenden Ansprüche, mit dem Schritt:
- Bereitstellung mindestens eines Substratkörpers (40-43) in mindestens einer der Kultivierungsphasen (20-23).

10. Verfahren nach Anspruch 9, bei dem der mindestens eine Substratkörper (40-43) in mindestens zwei aneinandergrenzenden Kultivierungsphasen (20-23) angeordnet ist.

11. Verfahren nach Anspruch 9 oder 10, bei dem der Substratkörper (40-43) einen Gelkörper, ein Netz, ein Filament, ein textiles Substrat und/oder eine Folie umfasst.

12. Verfahren nach mindestens einem der vorhergehenden Ansprüche, mit dem Schritt:
- Manipulation der biologischen Zellen, wobei wenigstens eines von einer Zellentnahme, einer Zellgruppierung oder einer Zellzufuhr vorgesehen ist.

13. Verfahren nach mindestens einem der vorhergehenden Ansprüche, bei dem die Kultivierungseinrichtung (100) eine Innenform aufweist, die nicht formgebend für die Gestalt der mindestens einen Grenzfläche (30-32) ist.

14. Verfahren nach mindestens einem der vorhergehenden Ansprüche, bei dem die Kultivierung der biologischen Zellen eine Bildung eines Zellverbandes oder Zellaggregates umfasst.

## Claims

1. A process for the cell cultivation of biological cells, except of human embryonic stem cells, on at least one curved boundary surface (30-32) between at least two different, non-solid cultivation phases (20-23) in a culture container (10) of a cultivation apparatus (100), with the steps of:
- cultivation phase adjustment so that predetermined cultivation conditions are given, wherein two liquid non-mixable cultivation phases (20-23) are adjusted in the cultivation apparatus (100), wherein at least one inner cultivation phase is completely enclosed by an outer cultivation phase, and
- cultivation of the biological cells on the at least one boundary surface (30, 32),
**characterized in that**
- the cultivation phases are arranged in the culture container (10), wherein firstly the outer cultivation phase (20) is filled into the culture container (10) and subsequently the inner cultivation phase (21) is injected and the cultivation phase adjustment in the culture container (10) is conducted before or during a deposition of the cells (1) at the at least one boundary surface (30-32),
- the cultivation phase adjustment comprises an influencing of the geometric properties of the cultivation phases (20-23), so that the at least one boundary surface (30-32) between the cultivation phases (20-23) gets a predetermined shape, and an arrangement of the cultivation phases (20-23) relative to each other, so that the boundary surface (30-32) has a predetermined extension, wherein additive substances, with which the mass density of the cultivation phases (20-23) is changed and the at least one boundary surface (30-32) is positioned and shape-stabilized in the cultivation apparatus (100), are introduced into the cultivation phases (20-23) in the cultivation apparatus (100) using an injection device (50), and
- the cultivation comprises a differentiation of the biological cells by signal factors and/or differentiation factors, which are included in at least one of the cultivation phases (20-23).

2. The process according to claim 1, wherein the cultivation phase adjustment comprises a formation of the cultivation phases (20-23) with predetermined cultivation media.

3. The process according to at least one of the previous claims, wherein the cultivation phase adjustment comprises a formation of the cultivation phases (20-23) with predetermined additive substances with which at least one of the viscosity and the miscibility of the cultivation phases is influenced.

4. The process according to at least one of the previous claims, with the step:
- supply of a gas to the biological cells.

5. The process according to at least one of the previous claims, with the step:
- providing a membrane (24) at the boundary surface (30-32) between two cultivation phases (20-23).

6. The process according to at least one of the previous claims, with the step:
- creating a phase transition in at least one of the cultivation phases (20-23).

7. The process according to at least one of the previous claims, with the step:
- fusion of at least two of the cultivation phases (20-23).

8. The process according to at least one of the previous claims, with the step:
- division of at least one of the cultivation phases (20-23) into separate cultivation phases.

9. The process according to at least one of the previous claims, with the step:
- providing at least one substrate body (40-43) in at least one of the cultivation phases (20-23).

10. The process according to Claim 9, wherein the at least one substrate body (40-43) is arranged in at least two cultivation phases (20-23) bordering each other.

11. The process according to claims 9 or 10, wherein the substrate body (40-43) comprises a gel body, a net, a filament, a textile substrate and/or a foil.

12. The process according to at least one of the previous claims, with the step:
- manipulation of the biological cells, wherein at least one is provided from a cell removal, a cell grouping or a cell supply.

13. The process according to at least one of the previous claims, wherein the cultivation apparatus (100) has an inner form which does not give the form for the shape of the at least one boundary surface (30-32).

14. The process according to at least one of the previous claims, wherein the cultivation of the biological cells comprises a formation of a cell composite or cell aggregate.

## Revendications

1. Procédé de culture cellulaire de cellules biologiques, à l'exception de cellules souches embryonnaires humaines, sur au moins une interface (30-32) courbe entre au moins deux phases de culture (20-23) différentes non solides dans un récipient de culture (10) d'un système de culture (100), présentant les étapes de :
- ajustement de phases de culture, de sorte que des conditions de culture prédéterminées soient créées, où, dans le système de culture (100), deux phases de culture (20-23) fluides non miscibles sont ajustées, où au moins une phase de culture interne est entièrement renfermée par une phase de culture externe, et
- culture des cellules biologiques sur l'au moins une interface (30-32),
**caractérisé en ce que**
- les phases de culture sont disposées dans le récipient de culture (10), où tout d'abord la phase de culture externe (20) est chargée dans le récipient de culture (10) et ensuite la phase de culture interne (21) est injectée et l'ajustement de phases de culture s'effectue dans le récipient de culture (10) avant ou pendant un dépôt des cellules (1) sur l'au moins une interface (30-32),
- l'ajustement de phases de culture comprend un effet des propriétés géométriques des phases de culture (20-23), de sorte que l'au moins une interface (30-32) entre les phases de culture (20-23) reçoive une forme prédéterminée, et comprend une disposition des phases de culture (20-23) les unes par rapport aux autres, de sorte que l'interface (30-32) présente une étendue prédéterminée, où des additifs sont injectés avec un système d'injection (50) dans les phases de culture (20-23) dans le système de culture (100), avec lesquels la densité de masse des phases de culture (20-23) est modifiée et l'au moins une interface (30-32) est positionnée dans le système de culture (100) et est stabilisée dans sa forme, et
- la culture comprend une différenciation des cellules biologiques par des facteurs de signal et/ou des facteurs de différenciation, qui sont contenus dans au moins l'une des phases de culture (20-23).

2. Procédé selon la revendication 1, dans lequel l'ajustement de phases de culture comprend une formation des phases de culture (20-23) avec des milieux de culture prédéterminés.

3. Procédé selon au moins l'une des revendications précédentes, dans lequel l'ajustement de phases de culture comprend une formation des phases de culture (20-23) avec des additifs prédéterminés, qui ont un effet sur au moins l'une de la viscosité et de la miscibilité des phases de cultures.

4. Procédé selon au moins l'une des revendications précédentes, présentant l'étape de :
- apport d'un gaz aux cellules biologiques.

5. Procédé selon au moins l'une des revendications précédentes, présentant l'étape de :
- mise à disposition d'une membrane (24) sur l'interface (30-32) entre deux phases de culture (20-23).

6. Procédé selon au moins l'une des revendications précédentes, présentant l'étape de :
- génération d'une transition de phase dans au moins l'une des phases de culture (20-23).

7. Procédé selon au moins l'une des revendications précédentes, présentant l'étape de :
- fusion d'au moins deux des phases de culture (20-23).

8. Procédé selon au moins l'une des revendications précédentes, présentant l'étape de :
- séparation d'au moins l'une des phases de culture (20-23) en phases de culture séparées.

9. Procédé selon au moins l'une des revendications précédentes, présentant l'étape de :
- mise à disposition d'au moins un corps de substrat (40-43) dans au moins l'une des phases de culture (20-23).

10. Procédé selon la revendication 9, dans lequel l'au moins un corps de substrat (40-43) est disposé dans au moins deux phases de culture (20-23) adjacentes.

11. Procédé selon la revendication 9 ou 10, dans lequel le corps de substrat (40-43) comprend un corps en gel, un filet, un filament, un substrat textile et/ou une feuille.

12. Procédé selon au moins l'une des revendications précédentes, présentant l'étape de :
- manipulation des cellules biologiques, où au moins l'un parmi un prélèvement de cellule, un groupement de cellule ou un apport de cellule est prévu.

13. Procédé selon au moins l'une des revendications précédentes, dans lequel le système de culture (100) présente une forme intérieure qui ne façonne pas la configuration de l'au moins une interface (30-32).

14. Procédé selon au moins l'une des revendications précédentes, dans lequel la culture des cellules biologiques comprend une formation d'un réseau de cellules ou d'un agrégat de cellules.
